# EUROPÄISCHE PATENTSCHRIFT

(11) **EP 1 637 178 B1**
(45) Veröffentlichungstag und Bekanntmachung des Hinweises auf die Patenterteilung: **20.02.2008**
(21) Anmeldenummer: 05018026.4
(22) Anmeldetag: 19.08.2005
(51) Int. Cl.: A61M 39/06, A61B 17/34

(54) **Ventil für medizinische Instrumente**
Valve for medical instrument
Soupape pour appareils médicaux

(30) Priorität: 17.09.2004 DE 102004045586
(43) Veröffentlichungstag der Anmeldung: 22.03.2006
(73) Patentinhaber: Karl Storz GmbH & Co. KG, 78532 Tuttlingen (DE)
(72) Erfinder: Eisenkolb, Peter, 78532 Tuttlingen (DE); Efinger, Andreas, 78604 Rietheim (DE)
(74) Vertreter: Hofmeister, Frank

(56) Entgegenhaltungen:
- US-A- 5 205 325
- US-A- 5 350 364
- US-A- 5 662 615
- US-A- 5 916 198

## Beschreibung

Die Erfindung betrifft ein Ventil für medizinische Instrumente, insbesondere endoskopische Instrumente, zum Abdichten eines in dem medizinischen Instrument ausgebildeten und zur Aufnahme mindestens eines weiteren medizinischen Instruments dienenden Kanals gegenüber einem distalseitigen Fluid, mit einem zumindest teilweise in den Kanal einsetzbaren Ventilgehäuse, wobei im Ventilgehäuse mindestens eine Fluidöffnung ausgebildet ist, und mit mindestens einem in dem Ventilgehäuse angeordneten, aus einem elastischen Material bestehenden Dichtkörper.

Derartige Ventile dienen dazu, den zur Aufnahme mindestens eines weiteren medizinischen Instruments dienenden Kanal in einem endoskopischen Instrument insbesondere dann abzudichten, wenn kein Instrument in den Kanal eingesteckt ist. Das Abdichten des Kanals erfolgt dabei gegenüber einem distalseitig anstehenden Fluid, bei dem es sich beispielsweise bei der Laparoskopie um ein zur Ausbildung des Pneumoperitoneums dienendes Gas oder auch, wie beispielsweise bei der Verwendung an einem Uretero-Renoskop, um eine Flüssigkeit handeln kann.

Zur Ausbildung derartiger Ventile ist es beispielsweise aus der Praxis bekannt, den in dem Ventilgehäuse angeordneten Dichtkörper als sogenannte "Duckbill" Dichtung auszubilden, bei der zwei zum distalseitigen Ende des Ventilgehäuses weisende Dichtlippen schnabelförmig aneinander anliegen. Diese mit derartigen Dichtkörpern ausgebildeten Ventile weisen in der Praxis unter anderem den Nachteil auf, dass ein Instrument nur von einer Seite, nämlich von proximal, durch die Dichtlippen geführt werden kann.

Ein gattungsgemäßes Ventil für medizinische Instrumente ist beispielsweise aus der DE 101 48 572 A1 bekannt. Zur Erhöhung der Dichtkraft des aus einem elastischen Material bestehenden Dichtkörper ist bei dieser bekannten Konstruktion möglich, die aneinander anliegenden Dichtkörper über die Fluidöffnung von außen mit einem Druckmedium, insbesondere Luft, zu beaufschlagen. Die Dichtkörper dieser bekannten Ventilkonstruktion gewährleisten zwar eine gute Abdichtung, jedoch bedeutet die Zufuhr eines externen Druckmediums einen hohen technischen und platzintensiven Aufwand, der insbesondere bei der Verwendung in endoskopischen Instrumenten kaum zu verwirklichen ist.

Aus der DE 693 13 504 T2 ist eine weitere Ventilanordnung bekannt, bei der der in dem Gehäuse festlegbare Dichtkörper so ausgebildet ist, dass der Dichtkörper über eine von außen auf den Dichtkörper einwirkende Einrichtung, wie beispielsweise eine Klemmschelle, so vorspannbar ist, dass der Dichtkörper den zur Aufnahme des einzusetzenden medizinischen Instruments dienenden Kanal bei nicht eingesetztem Instrument abdichtet. Neben dem komplizierten Aufbau dieser bekannten Ventilanordnung mit auf der Innenseite des Dichtkörpers ausgebildeten Führungselementen ist die Verwendung der von außen auf den Dichtkörper wirkenden Vorspanneinrichtung nachteilig, da einerseits diese Einrichtung den Aufbau dieses Ventil weiter verkompliziert und andererseits die Dichtwirkung des Ventils ausschließlich abhängig ist von der im Gebrauch einem Verschleiß unterliegenden Spannwirkung dieser Einrichtung.

Die US-A-5 662 615 offenbart ein Ventil mit einem Dichtkörper der proximal-und distalseitig einen umlaufenden Flansch aufweist, wobei der Dichtkörper mittels beider Flansche am Ventilgehäuse befestigt ist.

Davon ausgehend liegt der Erfindung die **Aufgabe** zugrunde, ein Ventil für medizinische Instrumente der eingangs genannten Art zu schaffen, dass bei einfachem Aufbau eine zuverlässige automatische Dichtwirkung gewährleistet.

Diese Aufgabenstellung ist erfindungsgemäß durch den kennzeichnenden Teil des ersten Anspruchs gelöst.

Durch die erfindungsgemäße Ausbildung ist es erstmalig möglich, ein in der Art eines Rückschlagventils arbeitendes selbstschließendes Ventil zu schaffen. Das Fluid dessen, Austritt durch den Einsatz des erfindungsgemäßen Ventils verhindert werden soll, sorgt seinerseits durch den von außen auf den Dichtkörper wirkenden Druck dafür, dass das Ventil zumindest dann geschlossen ist, wenn kein medizinisches Instrument in dem Kanal angeordnet ist.

Gemäß einer praktischen Ausführungsform der Erfindung wird vorgeschlagen, dass die mindestens eine Fluidöffnung als in Axialrichtung des Ventilgehäuses verlaufender Schlitz ausgebildet ist. Um eine rasche und gleichmäßige Fluid- und somit Druckverteilung zu gewährleisten, sind im Ventilgehäuse vorteilhafterweise mehrere, vorzugsweise drei, um den Umfang des Ventilgehäuses verteilt angeordnete, bis zum distalseitigen Ende des Ventilgehäuses reichende Schlitze als Fluidöffnungen ausgebildet.

Damit die Dichtwirkung des Dichtkörper nicht durch den distalseitig am Dichtkörper anliegenden Fluiddruck geschwächt oder gar aufgehoben werden kann, wird mit einer bevorzugten Ausführungsform der Erfindung vorgeschlagen, dass die mit dem Fluid in Kontakt stehende Fläche der Außenseite des Dichtkörpers größer ist als die mit dem Fluid beaufschlagte distalseitige Innenfläche des Dichtkörpers.

Zur Ausbildung des Dichtkörpers wird gemäß einer praktischen Ausführungsform der Erfindung vorgeschlagen, dass an der Innenseite des in dem Ventilgehäuse festlegbaren Dichtkörpers zwei sich gegenüberliegend angeordnete Dichtlippen ausgebildet sind, die in der Schließstellung den Kanal abdichtend zumindest linienförmig aneinander anliegen.

Die die eigentliche Abdichtung des Dichtkörpers bewirkenden Dichtlippen sind vorteilhafterweise einstückig mit dem Material des Dichtkörpers ausgebildet und bestehen aus dem selben Material wie der Dichtkörper.

Gemäß einer besonders einfach zu fertigenden Form der Dichtlippen sind diese im wesentlichen zur Mitte des Ventilgehäuses weisend im Querschnitt konvex ausgebildet und weisen beidseitig Anlaufschrägen auf, um das Einschieben eines medizinischen Instruments von beiden Seiten des Dichtkörpers zu ermöglichen. Neben dieser konvex-symmetrischen Ausbildung der Dichtlippen sind selbstverständlich auch andere, auch nicht-symmetrische Ausgestaltungen möglich.

Insbesondere, um die Dichtwirkung des Ventils bei in den Kanal eingeschobenem medizinischen Instrument sicherzustellen, wird mit der Erfindung weiterhin vorgeschlagen, dass proximalseitig vor dem Dichtkörper zumindest ein weiteres Dichtelement im Ventilgehäuse festlegbar ist. Selbstverständlich ist es auch möglich, das zusätzliche Dichtelement einstückig mit Dichtkörper auszubilden oder beispielsweise durch Verkleben fest mit dem Dichtkörper zu verbinden.

Gemäß einer praktischen Ausführungsform zur Ausbildung dieses zusätzlichen Dichtelements ist dieses als mit einer im wesentlichen zentral angeordneten Öffnung versehene Scheibendichtung ausgebildet, wobei der Durchmesser der in der Scheibendichtung ausgebildeten Öffnung kleiner ist als der Außendurchmesser eines durch diese Öffnung zu steckenden medizinischen Instruments, um eine fluiddichte Anlage der Dichtscheibe am Instrumentenschaft zu gewährleisten.

Um ein fluiddichtes und lagefestes Einsetzen des Ventilgehäuses in den Kanal des endoskopischen Instruments zu gewährleisten, wird mit der Erfindung weiterhin vorgeschlagen, dass auf der Außenseite des Ventilgehäuses mindestens ein Dichtelement angeordnet ist. Dieses auf der Außenseite des Ventilgehäuses angeordnete Dichtelement ist vorteilhafterweise als ein in einer im Ventilgehäuse ausgebildeten Ringnut festlegbarer Dichtring ausgebildet, insbesondere als aus Silikon gefertigter O-Ring.

Schließlich wird mit der Erfindung vorgeschlagen, dass am Ventilgehäuse zumindest proximalseitig Anlaufschrägen ausgebildet sind, durch die das Einführen eines medizinischen Instruments in den Kanal bzw., das Ventil erleichtert wird.

Weitere Merkmale und Vorteile der Erfindung ergeben sich anhand der zugehörigen Zeichnung, in der ein Ausführungsbeispiel eines erfindungsgemäßen Ventils für medizinische Instrumente nur beispielhaft dargestellt ist. In der Zeichnung zeigt:
- Fig. 1: eine perspektivische Ansicht eines erfindungsgemäßen Ventils;
- Fig. 2: einen Längsschnitt durch das Ventil gemäß Fig. 1;
- Fig. 3: eine perspektivische Ansicht des Dichtkörpers gemäß Fig. 2;
- Fig. 4: einen Längsschnitt durch den Dichtkörper gemäß Fig. 3 und
- Fig. 5: eine vergrößerte Darstellung des Dichtelements gemäß Fig. 2 im Längsschnitt.

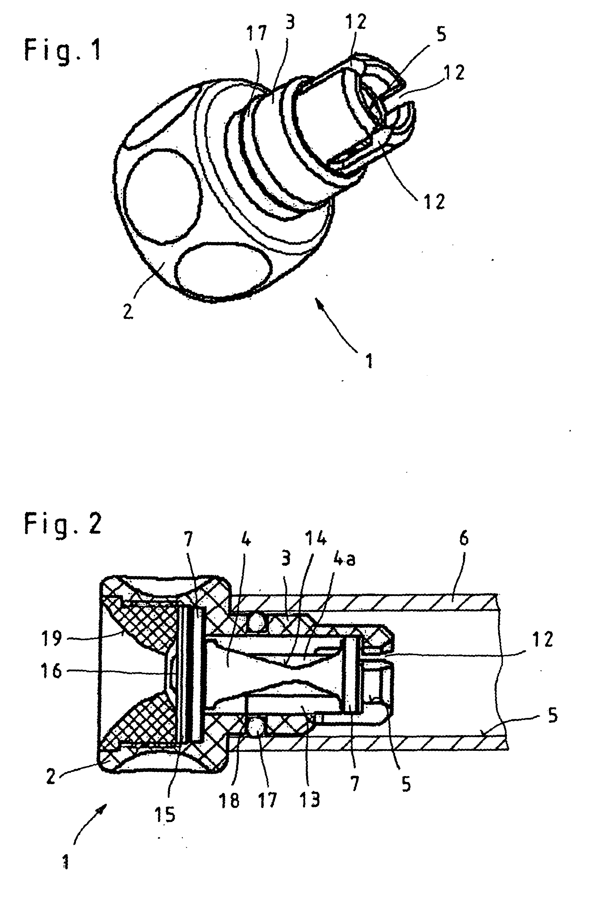

Die Abbildungen Fig. 1 und 2 zeigen ein Ventil, das im wesentlichen aus einem Ventilgehäuse 1 mit einem proximalseitigen Gehäusekopf 2 und einem sich daran anschließenden Gehäuseschaft 3 sowie einem überwiegend im Gehäuseschaft 3 des Ventilgehäuses 1 angeordneten Dichtkörper 4 besteht.

Das in einen Kanal 5 eines in Fig. 2 nur angedeutet dargestellten medizinischen Instruments 6 einsetzbare Ventil dient zum Abdichten des in dem medizinischen Instrument 6, insbesondere endoskopischen Instrument, ausgebildeten und zur Aufnahme mindestens eines weiteren medizinischen Instruments dienenden Kanals 5 gegenüber einem distalseitigen Fluid. Der zur Aufnahme des mindestens einen weiteren medizinischen Instruments dienende Kanal 5 setzt sich durch das Innere des Ventils fort, wie dies ebenfalls der Abbildung Fig. 2 zu entnehmen ist.

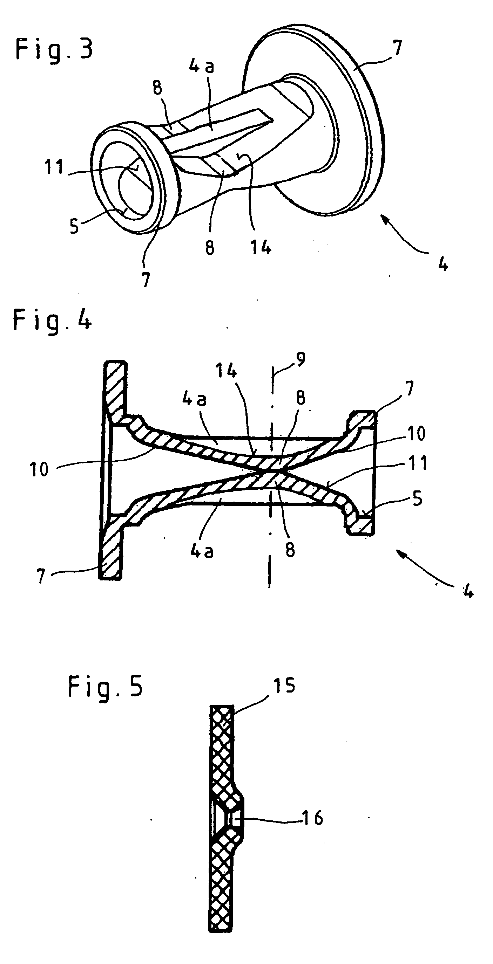

Der in dem Ventilgehäuse 1 angeordnete, aus einem elastischen Material gefertigte Dichtkörper 4 weist, wie insbesondere aus Fig. 3 und 4 ersichtlich, proximal- und distalseitig jeweils einen umlaufenden Flansch 7 auf, um den Dichtkörper 4 ortsfest im Ventilgehäuse 1 anordnen zu können, wobei die Flansche 7 entgegen der Darstellung gemäß Fig. 3 auch eckig ausgebildet sein können.

Die eigentliche Dichtfunktion des Dichtkörpers 4 übernehmen zwei sich gegenüberliegend angeordnete, an der Innenseite des Dichtkörpers 4 ausgebildete Dichtlippen 8, die in der in Fig. 4 dargestellten Schließstellung des Ventils den Kanal 5 abdichtend zumindest linienförmig aneinander anliegen. Zur Versteifung des Dichtkörpers 4 und insbesondere der Dichtlippen 8 weist der Dichtkörper 4 im Bereich der Dichtlippen 8 außen angeordnete Versteifungsstege 4a auf, wie diese insbesondere der Darstellung gemäß Fig. 3 zu entnehmen sind.

Bei der dargestellten Ausführungsform sind die Dichtlippen 8 zur Mitte des Ventilgehäuses 1 weisend im Querschnitt im wesentlichen konvex ausgebildet. Für die Dichtwirkung der Dichtlippen 8 des Dichtkörpers 4 spielt es keine Rolle, ob die Dichtlippen 8 symmetrisch zu einer Symmetrieachse 9 ausgebildet sind oder nicht. Um das Einführen des in den Kanal 5 einzusetzenden medizinischen Instruments zu ermöglichen, weisen die Dichtlippen 8 beidseitig Anlaufschrägen 10 auf. Durch die beidseitige Anordnung der Anlaufschrägen 10 ist es möglich, das einzuführende medizinische Instrument oder einen Führungsdraht sowohl von proximal als auch von distal in das Ventil einzuschieben.

Da die aneinander anliegenden Dichtlippen 8 des Dichtkörpers 4 durch den distalseitig an der Innenfläche 11 des Dichtkörpers 4 anliegenden Fluiddruck nach außen und somit in Richtung der Offenstellung des Ventils gedrückt werden, sind im Ventilgehäuse 1 Fluidöffnungen 12 ausgebildet, über die das distalseitige Fluid in einen Zwischenraum 13 zwischen der Außenseite 14 des Dichtkörpers 4 und der Innenseite des Ventilgehäuses 1 strömen kann. Dieses in den Zwischenraum 13 geströmte Fluid bewirkt einen im wesentlichen quer zur Längsachse des Dichtkörpers 4 nach innen auf den Dichtkörper 4 wirkenden Druck, durch den die Dichtlippen 8 in ihre Schließstellung gepresst werden.

Die Fluidöffnungen 12, die vorzugsweise als bis zum distalseitigen Ende des Ventilgehäuses 1 reichende Schlitze ausgebildet sind, sind insbesondere der perspektivischen Ansicht des Ventils gemäß Fig. 1 zu entnehmen. Bei der dargestellten Ausführungsform sind drei Fluidöffnungen 12 um den Umfang des Ventilgehäuses 1 verteilt angeordnet, wodurch ein schneller und gleichmäßiger Druckaufbau im Zwischenraum 13 gewährleistet wird. Selbstverständlich ist es auch möglich, mehr oder weniger als drei Fluidöffnungen 12 in dem Ventilgehäuse 1 auszubilden.

Um sicherzustellen, dass der über das Fluid von außen auf die Dichtlippen 8 aufgebrachte Druck immer für eine ausreichende Dichtwirkung sorgt, ist die mit dem Fluid in Kontakt stehende Fläche der Außenseite 14 des Dichtkörpers 4 so ausgelegt, dass sie zumindest gleich groß ist wie die mit dem Fluid beaufschlagte distalseitige Innenfläche 11 des Dichtkörpers 4. Vorzugsweise ist die mit dem Fluid in Kontakt stehende Fläche der Außenseite 14 des Dichtkörpers 4 aber größer als die mit dem Fluid beaufschlagte distalseitige Innenfläche 11 des Dichtkörpers 4.

Wie weiterhin aus der Schnittdarstellung gemäß Fig. 2 ersichtlich, ist bei dem dar gestellten Ausführungsbeispiel eines Ventils proximalseitig vor dem zuvor beschriebenen Dichtkörper 4 ein weiteres Dichtelement 15 angeordnet, welches in diesem Fall als mit einer zentralen Öffnung 16 versehene Scheibendichtung ausgebildet ist. Dieses in Fig. 5 dargestellte zusätzliche Dichtelement 15 dient zum Abdichten des Kanals 5 bei in den Kanal 5 eingesetztem medizinischen Instrument. Zu diesem Zweck ist der Durchmesser der in der Scheibendichtung ausgebildeten Öffnung 16 so bemessen, dass dieser kleiner ist als der Außendurchmesser des Schaftes des in den Kanal 5 einzusetzenden medizinischen Instruments.

Auf der Außenseite des Ventilgehäuses 1 ist ein weiteres Dichtelement 17 angeordnet, das zum fluiddichten Abdichten gegenüber dem medizinischen Instrument 6 dient, in dessen Kanal 5 das Ventil einsetzbar ist. Um ein Verrutschen des vorzugsweise als Ringdichtung ausgebildeten Dichtelements 17 zu verhindern, ist im Ventilgehäuse 1 eine Ringnut 18 ausgebildet, in der der Dichtring positionsgenau festlegbar ist. Der Dichtring ist vorteilhafterweise als aus Silikon gefertigter O-Ring ausgebildet. Zusätzlich zu der Dichtfunktion kann das auf der Außenseite des Ventilgehäuses 1 angeordnete Dichtelement 17 auch als die Einstecktiefe des Ventils in den Kanal 5 des medizinischen Instruments 6 begrenzender Anschlag verwendet werden sowie aufgrund seiner Elastizität eine Rastfunktion ausüben.

Schließlich weist das Ventilgehäuse 1 zumindest proximalseitig Anlaufschrägen 19 auf, die das Einsetzen des in den Kanal 5 einführbaren medizinischen Instruments deutlich erleichtern, da über die Anlaufschrägen 19 eine Zentrierung des Instruments zur Kanalmitte erreicht wird.

Das dargestellte Ventil zeichnet sich neben dem unkomplizierten Aufbau dadurch aus, dass es quasi die Funktion eines Rückschlagventils aufweist, da die Dichtwirkung des Ventils über dasselbe Fluid erfolgt, gegen dessen Austritt das Ventil in den Instrumentenkanal 5 eingesetzt wird. Im Gegensatz zu einem Rückschlagventil ist das dargestellte und beschriebene Ventil jedoch zweiseitig benutzbar.

### Bezugszeichenliste

- 1: Ventilgehäuse
- 2: Gehäusekopf
- 3: Gehäuseschaft
- 4: Dichtkörper
- 4a: Versteifungssteg
- 5: Kanal
- 6: medizinisches Instrument
- 7: Flansch
- 8: Dichtlippe
- 9: Symmetrieachse
- 10: Anlaufschräge
- 11: Innenfläche
- 12: Fluidöffnung
- 13: Zwischenraum
- 14: Außenseite
- 15: Dichtelement
- 16: Öffnung
- 17: Dichtelement
- 18: Ringnut
- 19: Anlaufschräge

## Patentansprüche

1. Ventil für medizinische Instrumente, insbesondere endoskopische Instrumente, zum Abdichten eines in dem medizinischen Instrument (6) ausgebildeten und zur Aufnahme mindestens eines weiteren medizinischen Instruments dienenden Kanals (5) gegenüber einem distalseitigen Fluid, mit einem zumindest teilweise in den Kanal (5) einsetzbaren Ventilgehäuse (1), wobei im Ventilgehäuse (1) mindestens eine Fluidöffnung (12) ausgebildet ist, und mit mindestens einem in dem Ventilgehäuse (1) angeordneten, aus einem elastischen Material bestehenden Dichtkörper (4), wobei die Fluidöffnung (12) so ausgebildet ist, dass das distalseitige Fluid aus dem Kanal (5) so an die Außenseite (14) des Dichtkörpers (4) leitbar ist, dass das distalseitige Fluid den Dichtkörper (4) abdichtend im wesentlichen quer zur Längsrichtung des Dichtkörpers (4) nach innen verformt, wobei an der Innenseite des Dichtkörpers zwei sich gegenüberliegend angeordnete Dichtlippen (8) ausgebildet sind,
**dadurch gekennzeichnet,**
**dass** der Dichtkörper (4) im Bereich der Dichtlippen (8) außen angeordnete Versteifungsstege (4a) aufweist, der Dichtkörper proximal- und distalseitig jeweils einen umlaufenden Flansch aufweist, wobei der Dichtkörper (4) über einen der angeformten Flansche (7) ortsfest im Ventilgehäuse (1) festlegbar ist.

2. Ventil nach Anspruch 1, **dadurch gekennzeichnet, dass** die mindestens eine Fluidöffnung (12) als in Axialrichtung des Ventilgehäuses (1) verlaufender Schlitz ausgebildet ist.

3. Ventil nach Anspruch 2, **dadurch gekennzeichnet, dass** im Ventilgehäuse (1) mehrere, vorzugsweise drei, um den Umfang des Ventilgehäuses (1) verteilt angeordnete, bis zum distalseitigen Ende des Ventilgehäuses (1) reichende Schlitze ausgebildet sind.

4. Ventil nach einem der Ansprüche 1 bis 3, **dadurch gekennzeichnet, dass** die mit dem Fluid in Kontakt stehende Fläche der Außenseite (14) des Dichtkörpers (4) größer ist als die mit dem Fluid beaufschlagte distalseitige Innenfläche (11) des Dichtkörpers (4).

5. Ventil nach einem der Ansprüche 1 bis 4, **dadurch gekennzeichnet, dass** die Dichtlippen (8) in der Schließstellung den Kanal (5) abdichtend zumindest linienförmig aneinander anliegen.

6. Ventil nach Anspruch 5, **dadurch gekennzeichnet, dass** die Dichtlippen (8) einstückig mit dem Dichtkörper (4) ausbildbar sind.

7. Ventil nach Anspruch 5 oder 6, **dadurch gekennzeichnet, dass** der Dichtkörper (4) und die Dichtlippen (8) aus dem selben Material bestehen.

8. Ventil nach einem der Ansprüche 5 bis 7, **dadurch gekennzeichnet, dass** die Dichtlippen (8) im Querschnitt im wesentlichen zur Mitte des Ventilgehäuses (1) weisend konvex ausgebildet sind.

9. Ventil nach einem der Ansprüche 5 bis 8, **dadurch gekennzeichnet, dass** an den Dichtlippen (8) beidseitig Anlaufschrägen (10) ausgebildet sind.

10. Ventil nach einem der Ansprüche 1 bis 9, **dadurch gekennzeichnet, dass** proximalseitig vor dem Dichtkörper (4) zumindest ein weiteres Dichtelement (15) im Ventilgehäuse (1) festlegbar ist.

11. Ventil nach Anspruch 10, **dadurch gekennzeichnet, dass** das mindestens eine zusätzliche Dichtelement (15) als mit einer im wesentlichen zentral angeordneten Öffnung (16) versehene Scheibendichtung ausgebildet ist.

12. Ventil nach Anspruch 11, **dadurch gekennzeichnet, dass** der Durchmesser der in der Scheibendichtung ausgebildeten Öffnung (16) kleiner ist als der Außendurchmesser eines durch diese Öffnung (16) zu steckenden medizinischen Instruments.

13. Ventil nach einem der Ansprüche 10 bis 12, **dadurch gekennzeichnet, dass** das weitere Dichtelement (15) einstückig mit dem Dichtkörper (4) ausgebildet ist.

14. Ventil nach einem der Ansprüche 10 bis 12, **dadurch gekennzeichnet, dass** das weitere Dichtelement (15), insbesondere durch Verkleben, fest mit dem Dichtkörper (4) verbunden ist.

15. Ventil nach einem der Ansprüche 1 bis 14, **dadurch gekennzeichnet, dass** auf der Außenseite des Ventilgehäuses (1) mindestens ein Dichtelement (17) angeordnet ist.

16. Ventil nach Anspruch 15, **dadurch gekennzeichnet, dass** das auf der Außenseite des Ventilgehäuses (1) angeordnete Dichtelement (17) als in einer im Ventilgehäuse (1) ausgebildeten Ringnut (18) festlegbarer Dichtring ausgebildet ist.

17. Ventil nach Anspruch 16, **dadurch gekennzeichnet, dass** der Dichtring ein aus Silikon gefertigter O-Ring ist.

18. Ventil nach einem der Ansprüche 1 bis 17, **dadurch gekennzeichnet, dass** am Ventilgehäuse (1) zumindest proximalseitig Anlaufschrägen (19) ausgebildet sind.

## Claims

1. Valve for medical instruments, in particular endoscopic instruments, with which a channel (5) formed in the medical instrument (6) and serving to receive at least one further medical instrument is sealed off from a fluid at the distal end, with a valve housing (1) that can be inserted at least partially into the channel (5), at least one fluid opening (12) being formed in the valve housing (1), and with at least one sealing body (4) arranged in the valve housing (1) and made of an elastic material, the fluid opening (12) being formed such that the fluid at the distal end can be conveyed from the channel (5) to the outside (14) of the sealing body (4) in such a way that the fluid at the distal end deforms the sealing body (4) in a sealing manner inwards substantially transverse to the longitudinal direction of the sealing body (4), with two sealing lips (8) being arranged opposite each other on the inside of the sealing body, **characterized in that** the sealing body (4) has stiffening webs (4a) arranged on the outside in the area of the sealing lips (8), the sealing body has a peripheral flange in each case at its proximal end and at its distal end, and the sealing body (4) can be secured in a fixed position in the valve housing (1) via one of the integrally formed flanges (7).

2. Valve according to Claim 1, **characterized in that** the at least one fluid opening (12) is formed as a slit that extends in the axial direction of the valve housing (1).

3. Valve according to Claim 2, **characterized in that** there are several, preferably three, slits formed in the valve housing (1), distributed about the circumference of the valve housing (1) and reaching as far as the distal end of the valve housing (1).

4. Valve according to one of Claims 1 to 3, **characterized in that** the surface of the outside (14) of the sealing body (4) in contact with the fluid is greater than the distal inside surface (11) of the sealing body (4) acted on by the fluid.

5. Valve according to one of Claims 1 to 4, **characterized in that** the sealing lips (8), in the closed position, bear at least linearly on each other to seal off the channel (5).

6. Valve according to Claim 5, **characterized in that** the sealing lips (8) can be designed in one piece with the sealing body (4).

7. Valve according to Claim 5 or 6, **characterized in that** the sealing body (4) and the sealing lips (8) are made of the same material.

8. Valve according to one of Claims 5 to 7, **characterized in that**, in cross section, the sealing lips (8) are of a substantially convex design pointing to the centre of the valve housing (1).

9. Valve according to one of Claims 5 to 8, **characterized in that** run-up slopes (10) are formed on the sealing lips (8) at both ends.

10. Valve according to one of Claims 1 to 9, **characterized in that** at least one further sealing element (15) can be secured in the valve housing (1) proximally in front of the sealing body (4)

11. Valve according to Claim 10, **characterized in that** the at least one additional sealing element (15) is designed as a disc seal provided with a substantially central opening (16).

12. Valve according to Claim 11, **characterized in that** the diameter of the opening (16) formed in the disc seal is smaller than the external diameter of a medical instrument to be inserted through this opening (16).

13. Valve according to one of Claims 10 to 12, **characterized in that** the further sealing element (15) is designed in one piece with the sealing body (4).

14. Valve according to one of Claims 10 to 12, **characterized in that** the further sealing element (15) is connected fixedly to the sealing body (4), in particular by gluing.

15. Valve according to one of Claims 1 to 14, **characterized in that** at least one sealing element (17) is arranged on the outside of the valve housing (1).

16. Valve according to Claim 15, **characterized in that** the sealing element (17) arranged on the outside of the valve housing (1) is designed as a sealing ring that can be secured in an annular groove (18) formed in the valve housing (1).

17. Valve according to Claim 16, **characterized in that** the sealing ring is an O-ring made of silicone.

18. Valve according to one of Claims 1 to 17, **characterized in that** run-up slopes (19) are formed on the valve housing (1) at least at the proximal end.

## Revendications

1. Soupape pour instruments médicaux, notamment des instruments endoscopiques, destiné à rendre étanche à l'encontre d'un fluide côté distal, un canal (5) qui est formé dans l'instrument médical (6) et sert à recevoir au moins un autre instrument médical, la soupape comprenant un corps de soupape (1) qui peut être inséré au moins partiellement dans le canal (5), au moins une ouverture de fluide (12) étant réalisée dans le corps de soupape (1), et comprenant également au moins un corps d'étanchéité (4) réalisé en un matériau élastique et agencé dans le corps de soupape (1), l'ouverture de fluide (12) étant configurée de manière à ce que le fluide côté distal en provenance du canal (5) puisse être dirigé vers le côté extérieur (14) du corps d'étanchéité (4) de façon telle que le fluide côté distal déforme le corps d'étanchéité (4) vers l'intérieur, essentiellement de manière transversale par rapport à la direction longitudinale du corps d'étanchéité (4), en le rendant étanche, deux lèvres d'étanchéité (8) mutuellement opposées étant formées sur le côté intérieur du corps d'étanchéité,
**caractérisée en ce que** le corps d'étanchéité (4) présente, dans la zone des lèvres d'étanchéité (8), des nervures de renfort (4a) agencées à l'extérieur, et le corps d'étanchéité présente un flasque périphérique respectivement du côté proximal et du côté distal, le corps d'étanchéité (4) pouvant, par l'intermédiaire de l'un des flasques (7) qui y sont formés, être fixé en position localisée dans le corps de soupape (1).

2. Soupape selon la revendication 1, **caractérisée en ce que** ladite au moins une ouverture de fluide (12) est réalisée en tant que fente s'étendant dans la direction axiale du corps de soupape (1).

3. Soupape selon la revendication 2, **caractérisée en ce que** dans le corps de soupape (1) sont réalisées plusieurs, de préférence trois fentes, qui sont agencées de manière répartie le long de la périphérie du corps de soupape (1), et s'étendent jusqu'à l'extrémité distale du corps de soupape (1).

4. Soupape selon l'une des revendications 1 à 3, **caractérisée en ce que** la surface du côté extérieur (14) du corps d'étanchéité (4), qui est en contact avec le fluide, est plus grande que la surface intérieure distale (11) du corps d'étanchéité (4), qui est sollicitée par le fluide.

5. Soupape selon l'une des revendications 1 à 4, **caractérisée en ce que** les lèvres d'étanchéité (8), dans la position de fermeture, s'appliquent l'une sur l'autre au moins sur une zone en forme de ligne, en rendant étanche le canal (5).

6. Soupape selon la revendication 5, **caractérisée en ce que** les lèvres d'étanchéité (8) peuvent être réalisées d'un seul tenant avec le corps d'étanchéité (4).

7. Soupape selon la revendication 5 ou 6, **caractérisée en ce que** corps d'étanchéité (4) et les lèvres d'étanchéité (8) sont réalisés dans le même matériau.

8. Soupape selon l'une des revendications 5 à 7, **caractérisée en ce que** les lèvres d'étanchéité (8), en section transversale, sont réalisées convexes en étant sensiblement dirigées vers le centre du corps de soupape (1).

9. Soupape selon l'une des revendications 5 à 8, **caractérisée en ce que** sur les lèvres d'étanchéité (8) sont formées, des deux côtés, des rampes inclinées d'entrée (10).

10. Soupape selon l'une des revendications 1 à 9, **caractérisée en ce que** du côté proximal, avant le corps d'étanchéité (4), au moins un autre élément d'étanchéité (15) peut être fixé dans le corps de soupape (1).

11. Soupape selon la revendication 10, **caractérisée en ce que** ledit au moins un élément d'étanchéité supplémentaire (15) est réalisé en tant que joint d'étanchéité en forme de disque pourvu d'une ouverture (16) agencée sensiblement de manière centrale.

12. Soupape selon la revendication 11, **caractérisée en ce que** le diamètre de l'ouverture (16) formée dans le joint d'étanchéité en forme de disque, est inférieur au diamètre extérieur d'un instrument médical devant être inséré à travers cette ouverture (16).

13. Soupape selon l'une des revendications 10 à 12, **caractérisée en ce que** ledit autre élément d'étanchéité (15) est réalisé d'un seul tenant avec le corps d'étanchéité (4).

14. Soupape selon l'une des revendications 10 à 12, **caractérisée en ce que** ledit autre élément d'étanchéité (15) est relié de manière fixe, notamment par collage, au corps d'étanchéité (4).

15. Soupape selon l'une des revendications 1 à 14, **caractérisée en ce que** sur le côté extérieur du corps de soupape (1) est agencé au moins un élément d'étanchéité (17).

16. Soupape selon la revendication 15, **caractérisée en ce que** l'élément d'étanchéité (17) agencé sur le côté extérieur du corps de soupape (1), est réalisé sous forme de bague d'étanchéité pouvant être fixée ou immobilisée dans une rainure annulaire (18) formée dans le corps de soupape (1).

17. Soupape selon la revendication 16, **caractérisée en ce que** la bague d'étanchéité est un joint torique fabriqué en silicone.

18. Soupape selon l'une des revendications 1 à 17, **caractérisée en ce que** sur le corps de soupape (1) sont formées, au moins du côté proximal, des rampes inclinées d'entrée (19).
